(19) Europäisches Patentamt
European Patent Office
Office européen des brevets

(11) EP 4 609 851 A1

(12) **EUROPEAN PATENT APPLICATION**
published in accordance with Art. 153(4) EPC

(43) Date of publication:
**03.09.2025 Bulletin 2025/36**

(21) Application number: **23882636.6**

(22) Date of filing: **24.10.2023**

(51) International Patent Classification (IPC):
***A61K 8/86*** (2006.01) ***A61K 8/37*** (2006.01)
***A61K 8/39*** (2006.01) ***A61Q 19/10*** (2006.01)
***C07C 69/33*** (2006.01) ***C08G 65/332*** (2006.01)

(52) Cooperative Patent Classification (CPC):
**A61K 8/37; A61K 8/39; A61K 8/45; A61K 8/86; A61Q 1/00; A61Q 19/10; C07C 69/33; C08G 65/332; C09K 23/42; C09K 23/52**

(86) International application number:
**PCT/JP2023/038339**

(87) International publication number:
**WO 2024/090431 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.10.2022 JP 2022173544**
**31.01.2023 JP 2023013486**

(71) Applicant: **Taiyo Kagaku Co., Ltd.**
**Mie 512-1111 (JP)**

(72) Inventors:
- **MATSUMOTO, Yoshiyuki**
  **Yokkaichi-shi, Mie, 512-1111 (JP)**
- **SAKANISHI, Yuichi**
  **Yokkaichi-shi, Mie, 512-1111 (JP)**
- **HIGUCHI, Tomonori**
  **Yokkaichi-shi, Mie, 512-1111 (JP)**

(74) Representative: **Vossius & Partner Patentanwälte Rechtsanwälte mbB**
**Siebertstrasse 3**
**81675 München (DE)**

(54) **POLYGLYCERYL FATTY ACID ESTER**

(57) A polyglycerol fatty acid ester consisting of a polyglycerol of which the ratio of a branched glycerol group represented by the following formula (1):

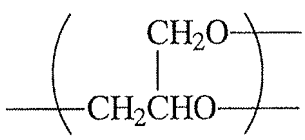

(1)

is from 14 to 36% and an average degree of polymerization is from 5 to 22, and a fatty acid having the number of carbon atoms of from 6 to 22, wherein the total content of glycerol and diglycerol in the polyglycerol is from 2 to 20% by mass. According to the present invention, a polyglycerol fatty acid ester which is excellent in solubility to an oil agent in wide temperature range can be provided, and the polyglycerol fatty acid ester can be suitably used in an application such as cosmetics.

EP 4 609 851 A1

## Description

### TECHNICAL FIELD

The present invention relates to a polyglycerol fatty acid ester, a composition containing the compound, and cosmetics containing these.

### BACKGROUND ART

A polyglycerol fatty acid ester is mainly utilized in the fields of foods and cosmetics. An example of exploitation in the field of cosmetics includes oily cleansing cosmetics in which the polyglycerol fatty acid ester is dissolved in an oil agent. Upon formulating into the oily cleansing cosmetics, a key factor includes solubility to the oil agent. When the solubility to the oil agent is poor, precipitations or turbidity disadvantageously occur, and cosmetics of which an appearance is impaired are disadvantageously produced.

A polyglycerol which is used in a hydrophilic group of the polyglycerol fatty acid ester is conventionally obtained by dehydrating and polymerizing a glycerol, and optionally purifying the glycerol by distillation, decolorization, deodorization, ion-exchange resin treatment or the like. The polyglycerol prepared by such a method causes turbidity or precipitation, when a polyglycerol fatty acid ester is synthesized and dissolved in an oil agent.

In addition, one of methods of increasing the solubility to an oil agent includes a method of lowering an HLB of a surfactant. A transparent solution can be transitorily formed by lowering an HLB, but the precipitation disadvantageously occurs in the storage for a long term or under low temperature range, so that a solution of the problems has been not achieved.

Patent Publication 1, for example, discloses a method of increasing the solubility to the oil agent by removing unreacted polyglycerol of the polyglycerol fatty acid ester as a technique for solving the problems.

### PRIOR ART REFERENCES

### PATENT PUBLICATIONS

Patent Publication 1: Japanese Patent Laid-Open No. Hei 7-173380

### SUMMARY OF THE INVENTION

### PROBLEMS TO BE SOLVED BY THE INVENTION

However, a step of removing the unreacted polyglycerol is required and the stability under a low temperature is not yet evaluated in Patent Publication 1, so that further improvements are required.

The problems to be solved by the present invention are to provide a polyglycerol fatty acid ester having an excellent solubility to an oil agent even under a low temperature such as -5°C.

### MEANS TO SOLVE THE PROBLEMS

The present invention relates to the following [1] to [3]:

[1] A polyglycerol fatty acid ester consisting of a polyglycerol of which the ratio of a branched glycerol group represented by the following formula (1):

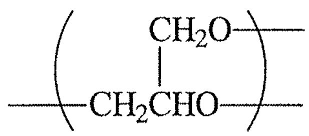

(1)

is from 14 to 36% and an average degree of polymerization is from 5 to 22, and a fatty acid having the number of carbon atoms of from 6 to 22, wherein the total content of glycerol and diglycerol in the polyglycerol is from 2 to 20% by mass.

[2] A cosmetic composition containing the polyglycerol fatty acid ester as defined in [1] and an oil agent.

[3] Cosmetics containing the cosmetic composition as defined in [2].

## EFFECTS OF THE INVENTION

According to the present invention, a polyglycerol fatty acid ester having an excellent solubility to an oil agent even under a low temperature such as -5°C can be provided. The solubility in the present invention is judged by a solubility under -5°C in the case where cetyl ethylhexanoate is used as an oil agent and the concentration of the polyglycerol fatty acid ester is 15% by mass, but the polyglycerol fatty acid ester of the present invention can also be used in the oil agent, concentration and temperature range other than the above.

## BRIEF DESCRIPTION OF THE DRAWINGS

[FIG. 1] FIG. 1 is a 13C-NMR spectrum of Preparation Example 1.

## MODES FOR CARRYING OUT THE INVENTION

As a result of intensively studying the above problems, the inventors found that the solubility to an oil agent is made excellent by allowing a polyglycerol which is a hydrophilic group of a polyglycerol fatty acid ester to have an appropriate branched structure and setting the total content of glycerol and diglycerol in the polyglycerol within the specific range, thereby completing the present invention. As to such a mechanism, it is assumed that a polyglycerol which is a hydrophilic group has an appropriate branched structure, whereby the hydrophilic groups are likely to interact with each other upon forming a reverse micelle structure. In addition, it is considered that the specified amount of ester in the glycerol and diglycerol consolidates the reverse micelle structure, to further increase the stability.

The polyglycerol fatty acid ester of the present invention is an ester of a polyglycerol of which the ratio of branched glycerol group represented by the above formula (1) is from 14 to 36% and an average degree of polymerization is from 5 to 22, and a fatty acid having the number of carbon atoms of from 6 to 22. An HLB of polyglycerol fatty acid ester of the present invention is preferably from 8 to 13, and more preferably from 9 to 13, from the viewpoint of inhibiting stickiness upon washing out.

The HLB as used herein refers to a value which is measured by the following Griffin's calculation method. A saponification value and a neutralization value are measured by a known method.

$$HLB = 20\,(1 - S / A)$$

S: Saponification value of polyglycerol fatty acid ester
A: Neutralization value of raw material fatty acid

In the polyglycerol of the polyglycerol fatty acid ester of the present invention, the ratio of the branched glycerol group is from 14 to 36%, the average degree of polymerization is from 5 to 22, and preferably from 5 to 15%, from the viewpoint of the solubility to the oil agent. In addition, the number of the branched glycerol group represented by the above formula (1) per polyglycerol molecule is preferably from 1 to 10 on average, and more preferably from 1 to 4 on average. Here, the ratio and the number of the branched glycerol group mentioned above can be adjusted by the raw material ratio at the time of manufacturing a polyglycerol. For example, in Examples described later, increasing the usage of the glycerol triglycidyl ether increases the ratio and the number of the branched glycerol group mentioned above, and decreasing the usage of the glycerol triglycidyl ether decreases the ratio and the number of the branched glycerol group mentioned above. Incidentally, the ratio and the number of the branched glycerol group mentioned above are measured by the method described in Examples described later.

The average degree of polymerization of the polyglycerol in the specification is an average degree of polymerization of the polyglycerol calculated from the hydroxyl value according to a terminal group analysis method, and is an average degree of polymerization calculated from (Formula 1) and (Formula 2).

Average degree of polymerization = (112.2 × 103 - 18 × hydroxyl value) / (74 × hydroxyl value - 56.1 × 103) (Formula 1)

Hydroxyl value = (a - b) × 28.05 / harvested amount of sample (g) (Formula 2)

a: Consumed amount of 0.5 N potassium hydroxide solution according to blank test (ml)
b: Consumed amount of 0.5 N potassium hydroxide solution according to main test (ml)

The hydroxyl value in the above (Formula 1) is calculated by (Formula 2) according to "Standard Methods for the Analysis of Fats, Oils and Related Materials (I), 1996, constituted by Japan Oil Chemists' Society" edited by a general incorporated association, Japan Oil Chemists' Society.

In addition, as to the polyglycerol according to the polyglycerol fatty acid ester of the present invention, total content of the glycerol and diglycerol is from 2 to 20% by mass, and preferably from 4 to 20% by mass, from the viewpoint of the solubility to the oil agent and degree of polymerization. The total content of the glycerol and diglycerol in the polyglycerol can be adjusted by control of the degree of purification by a column, a separate addition after purification or the like. In addition, the total content of the glycerol and diglycerol in the polyglycerol is measured by the method described in Examples mentioned later.

As to the fatty acid according to polyglycerol fatty acid ester of the present invention, the number of carbon atoms is from 6 to 22, preferably from 8 to 20, and more preferably from 8 to 18, from the viewpoint of a crystallinity in the oil agent. In addition, the number of carbon atoms may be from 6 to 12, from 8 to 12, from 14 to 22, from 14 to 20, from 14 to 18 and the like. Concretely, the fatty acid includes caprylic acid, isononanoic acid, capric acid, lauric acid, isostearic acid, oleic acid and the like.

The polyglycerol fatty acid ester of the present invention can be prepared by esterifying a polyglycerol with a fatty acid according to a general synthesis method. The polyglycerol fatty acid ester of the present invention can be suitably used as a cosmetic composition containing a polyglycerol fatty acid ester and an oil agent, because the polyglycerol fatty acid ester has excellent solubility to the oil agent. In addition, the polyglycerol fatty acid ester can be used in a known application as an application of a polyglycerol fatty acid ester such as the use in various foodstuff. Hereinafter, a preferable embodiment of a cosmetic composition using the polyglycerol fatty acid ester of the present invention is explained.

The cosmetic composition of the embodiment contains the polyglycerol fatty acid ester of the present invention and an oil agent.

The content of the polyglycerol fatty acid ester in the cosmetic composition of the embodiment is preferably from 3 to 30% by mass, more preferably from 5 to 20% by mass, and even more preferably from 10 to 15% by mass. Here, the polyglycerol fatty acid ester used includes at least the polyglycerol fatty acid ester of the present invention, but other polyglycerol fatty acid esters can be used in combination. The proportion of the polyglycerol fatty acid ester of the present invention in a polyglycerol fatty acid ester is preferably from 50 to 100% by mass, more preferably from 70 to 100% by mass, and even more preferably from 90 to 100% by mass. Incidentally, the content in the case where two or more kinds of polyglycerol fatty acid esters are used refers to a total amount of the polyglycerol fatty acid ester.

The oil agent in the cosmetic composition of the embodiment includes, but not particularly limited to, for example, hydrocarbon oil, ester oil, acylglycerol, ether oil, animal and vegetable oil, silicon oil and the like, and preferably hydrocarbon oil, ester oil, ether oil, vegetable oil, and more preferably ester oil.

The hydrocarbon oil includes, but not particularly limited to, for example, hydrogenated polyisobutene, liquid paraffin, light liquid isoparaffin, squalane, undecane, tridecane, isododecane and the like.

The ester oil includes, but not particularly limited to, for example, isononyl isononanoate, isostearyl isostearate, cetyl ethylhexanoate, ethylhexyl palmitate, octyldodecyl myristate, isopropyl myristate, propylheptyl caprylate, caprylyl caprylate, caprylyl caprate, cocoalkyl (caprylate / caprate), dicaprylyl carbonate and the like.

The acylglycerol includes, but not particularly limited to, for example, (caprylic / capric) glycerides, glyceryl tri(caprylate / caprate), triethylhexanoin, glyceryl triisostearate, glyceryl diisostearate and the like.

The ether oil includes, but not particularly limited to, for example, dicaprylyl ether and the like.

The animal and vegetable oil includes, but not particularly limited to, for example, beeswax, avocado oil, almond oil, olive oil, wheat germ oil, rice germ oil, rice bran oil, safflower oil, soybean oil, corn oil, rapeseed oil, palm oil, palm kernel oil, castor oil, sunflower oil, jojoba oil, macadamia nut oil, coconut oil, lanolin and the like, and preferably avocado oil, almond oil, olive oil, wheat germ oil, rice germ oil, rice bran oil, safflower oil, soybean oil, corn oil, rapeseed oil, castor oil, sunflower oil, jojoba oil, macadamia nut oil and the like.

The silicone oil includes, but not particularly limited to, for example, dimethicone, phenyl trimethicone, cyclomethicone, cyclopentasiloxane, diphenylsiloxy phenyl trimethicone and the like.

The content of the oil agent in the cosmetic composition of the embodiment is preferably from 70 to 97% by mass, more preferably from 80 to 95% by mass, and even more preferably from 85 to 90% by mass. Incidentally, the content of the oil agent in the case where two or more kinds of oil agents are used refers to a total amount of the oil agents.

In the cosmetic composition of the embodiment, ingredients which are generally used in cosmetics other than the above ingredients can be appropriately formulated depending upon the use or purpose thereof. These optional ingredients include, for example, water, surfactants, polyhydric alcohol, aqueous gelation agents, oily gelation agents, ultraviolet absorbents, powders, antioxidants, preservatives, perfumes, colorants, chelating agents, refreshing agents, thickening agents, plant extracts, vitamins, neutralizing agents, moisturizing agents, antiinflammatory agents, pH adjusting agents, amino acids and the like.

The surfactant includes, but not particularly limited to, for example, polyoxyethylene fatty acid ester, polyoxyethylene glycerol fatty acid ester, polyoxyethylene sorbitan fatty acid ester, polyoxyethylene alkyl ether, polyoxyethylene (hydrogenated) castor oil and the like.

The polyhydric alcohol includes, but not particularly limited to, for example, glycerol, diglycerol, ethylhexylglycerin, 1,3-butylene glycol (BG), propylene glycol (PG), propanediol, dipropylene glycol (DPG), pentylene glycol, 1,2-hexanediol, caprylyl glycol, 1,10-decanediol, isopentyldiol, sorbitol, mannitol, maltitol, xylitol and the like.

The cosmetic composition of the embodiment can be prepared by mixing the above ingredients according to a conventional method.

The cosmetic composition of the embodiment can be suitably used in various cosmetics. In other words, the present invention provides cosmetics containing the cosmetic composition of the embodiment. Here, the cosmetic composition of the embodiment may be directly used as cosmetics, or those further containing optional ingredients mentioned above can be made as cosmetics.

The cosmetics include oily cosmetics, emulsified cosmetics, bicontinuous type cosmetics and the like, and preferably oily cosmetics. For example, the cosmetics include cleansing cosmetics such as cleansing oil, cleansing balm, hot cleansing balm, cleansing gel, hot cleansing gel, cleansing gel pack, massage cosmetics such as massage oil, massage oil gel, massage scrub gel, hot massage gel, bath salts and the like.

The method of producing the cosmetics includes a method of producing cosmetics including the step of containing each of the above ingredients. Here, "step of containing each of the above ingredients" includes an embodiment of adding the previously prepared cosmetic composition, and additionally an embodiment of separately formulating each of the above ingredients and preparing.

## EXAMPLES

Hereinafter, the present invention will be particularly described by showing Examples and Comparative Examples, without intending to limit the scope of the present invention to the following Examples.

### Polyglycerol Production Example 1

To a three-necked flask equipped with Dimroth and a stirrer, 100 g of glycerol and an alkaline catalyst were added, and the temperature was elevated to 120°C. Glycerol triglycidyl ether in an amount of 28.26 g was added thereto dropwise at a rate of 10 g/hr, and after completion of addition dropwise, the mixture was reacted for additional 1 hour. Thereafter, the solution passed through an ion-exchange resin column, and unreacted glycerol was removed. The resulting fraction was concentrated to provide polyglycerol 1.

### Polyglycerol Production Example 2

To a three-necked flask equipped with Dimroth and a stirrer, 100 g of glycerol and an alkaline catalyst were added, and the temperature was elevated to 120°C. Glycerol triglycidyl ether in an amount of 47.1 g was added thereto dropwise at a rate of 10 g/hr, and after completion of addition dropwise, the mixture was reacted for additional 1 hour. Thereafter, the solution passed through an ion-exchange resin column, and unreacted glycerol was removed. The resulting fraction was concentrated to provide polyglycerol 2.

### Polyglycerol Production Example 3

To a three-necked flask equipped with Dimroth and a stirrer, 100 g of diglycerol and an alkaline catalyst were added, and the temperature was elevated to 140°C. Glycerol triglycidyl ether in an amount of 19.56 g was added thereto dropwise at a rate of 10 g/hr, and after completion of addition dropwise, the mixture was reacted for additional 1 hour. Thereafter, the solution passed through an ion-exchange resin column, and unreacted diglycerol was removed. The resulting fraction was concentrated to provide polyglycerol 3.

### Polyglycerol Production Example 4

To a three-necked flask equipped with Dimroth and a stirrer, 100 g of diglycerol and an alkaline catalyst were added, and the temperature was elevated to 140°C. Glycerol triglycidyl ether in an amount of 39.13 g was added thereto dropwise at a rate of 10 g/hr, and after completion of addition dropwise, the mixture was reacted for additional 1 hour. Thereafter, the solution passed through an ion-exchange resin column, and unreacted diglycerol was removed. The resulting fraction was concentrated to provide polyglycerol 4.

Polyglycerol Production Example 5

To a three-necked flask equipped with Dimroth and a stirrer, 100 g of diglycerol and an alkaline catalyst were added, and the temperature was elevated to 140°C. Glycerol triglycidyl ether in an amount of 78.26 g was added thereto dropwise at a rate of 10 g/hr, and after completion of addition dropwise, the mixture was reacted for additional 1 hour. Thereafter, the solution passed through an ion-exchange resin column, and unreacted diglycerol was removed. The resulting fraction was concentrated to provide polyglycerol 5.

Polyglycerol Production Example 6

To a three-necked flask equipped with Dimroth and a stirrer, 100 g of glycerol and an alkaline catalyst were added, and the temperature was elevated to 140°C. Glycerol triglycidyl ether in an amount of 141.3 g was added thereto dropwise at a rate of 10 g/hr, and after completion of addition dropwise, the mixture was reacted for additional 1 hour. Thereafter, the solution passed through an ion-exchange resin column, and unreacted glycerol was removed. The resulting fraction was concentrated to provide polyglycerol 6.

Polyglycerols 7 and 8

Additionally, "polyglycerol #750," manufactured by Sakamoto Yakuhin Kogyo Co., LTD. was used as polyglycerol 7, and "polyglycerol #500," manufactured by Sakamoto Yakuhin Kogyo Co., LTD. was used as polyglycerol 8.

Polyglycerol Production Example 9

To a three-necked flask equipped with Dimroth and a stirrer, 100 g of glycerol and an alkaline catalyst were added, and the temperature was elevated to 120°C. Glycerol triglycidyl ether in an amount of 28.26 g was added thereto dropwise at a rate of 10 g/hr, and after completion of addition dropwise, the mixture was reacted for additional 1 hour. Thereafter, the solution passed through an ion-exchange resin column twice, and unreacted glycerol was removed as much as possible. The resulting fraction was concentrated to provide polyglycerol 9.

Polyglycerol Production Examples 10 to 13

A glycerol or diglycerol was added to the polyglycerol 9 to adjust the contents as described in Table 1.

< Measurement of Ratio of Branched Glycerol and Number of Branched Glycerol per Molecule >

The ratio of branched glycerol and the number of branched glycerol per molecule were calculated as follows by 13C-NMR. The result is given in Table 1. In addition, 13C-NMR spectrum of polyglycerol 1 is shown in FIG. 1 as a representative example of 13C-NMR spectrum.

Pretreatment: 200 mg of polyglycerol was dissolved in 0.6 mL of heavy water
Apparatus used: 800 MHz 13C-NMR, "JNM-ECZ800R" manufactured by JEOL Ltd.
Cumulative number: 256 times
Measurement condition: temperature 30°C, measurement sequence single pulse 1H decoupling, pulse repetition time 7 seconds, and measurement is carried out using acetone as a standard peak (δ: 30.89 ppm)
Each peak range used for integral:

Total Integrals (TI): 60.0 - 83.0 ppm
Dendritic (D): 76.5 - 80.0 ppm

Method of Calculating Ratio of Branched Glycerol

TI shows integral values of all carbon atoms regarding polyglycerol, and D shows integral values of secondary carbon atoms regarding branched glycerol. The ratio of branched glycerol (%) is calculated from integral values of each peak mentioned above according to the following formula

$$\text{Ratio of branched glycerol (\%)} = (D \times 3 / TI) \times 100$$

Method of Calculating Number of Branched Glycerol per Molecule

The number of branched glycerol per molecule of polyglycerol is calculated according to the following formula

Number of branched glycerol per molecule = (D × 3 / TI) × average degree of polymerization

< Measurement of Content of Glycerol and Diglycerol >

The contents of glycerol and diglycerol in a polyglycerol was measured by making polyglycerol as a derivative according to trimethylsilylation, and carrying out separation and quantification of the polyglycerol derivative according to GC method (gas chromatograph method). The result is given in Table 1.

Apparatus used: GC-14B (SHIMADZU CORPORATION)
Column: OL-17 (50% phenyl methyl silicone, GL Sciences Inc.)
Column size: 0.5 m × 3 mm
Measurement temperature: 100 → 300°C
Heating rate: 10°C/min

[Table 1]

Table 1

| | Ratio of branched glycerol group (%) | Average degree of polymerization | Number of the branched glycerol group per molecule | A. amount of glycerol (% by mass) | B. amount of diglycerol (% by mass) | A+B (% by mass) |
|---|---|---|---|---|---|---|
| Polyglycerol 1 | 34.0 | 5.2 | 1.77 | 17.8 | 0.0 | 17.8 |
| Polyglycerol 2 | 31.1 | 6.2 | 1.93 | 10.3 | 0.0 | 10.3 |
| Polyglycerol 3 | 17.3 | 8.0 | 1.38 | 0.0 | 11.5 | 11.5 |
| Polyglycerol 4 | 26.2 | 12.3 | 3.22 | 0.0 | 4.8 | 4.8 |
| Polyglycerol 5 | 33.0 | 20.5 | 6.77 | 0.0 | 2.5 | 2.5 |
| Polyglycerol 6 | 55.0 | 24.3 | 13.37 | 3.2 | 0.0 | 3.2 |
| Polyglycerol 7 | 5.6 | 10.0 | 0.56 | 3.1 | 11.8 | 14.9 |
| Polyglycerol 8 | 4.7 | 6.0 | 0.28 | 0.1 | 6.7 | 6.8 |
| Polyglycerol 9 | 34.1 | 6.8 | 2.32 | 0.2 | 0.0 | 0.2 |
| Polyglycerol 10 | 32.1 | 6.3 | 2.02 | 4.9 | 0.0 | 4.9 |
| Polyglycerol 11 | 32.0 | 6.5 | 2.08 | 0.2 | 5.2 | 5.4 |
| Polyglycerol 12 | 25.6 | 5.4 | 1.38 | 25.2 | 0.0 | 25.2 |
| Polyglycerol 13 | 25.9 | 5.8 | 1.50 | 0.1 | 24.5 | 24.6 |

Preparation of Polyglycerol Fatty Acid Ester

Examples 1 to 14 and 16 to 17, and Comparative Examples 1 to 11 and 13 to 15

Polyglycerol fatty acid ester of each Example and Comparative Example was prepared as shown in Tables 2 to 4. A method of preparing Example 1 is more specifically explained as a representative example. To a four-necked flask equipped with a stirrer, 20.3 g of polyglycerol 1 obtained in Polyglycerol Production Example 1, 19.7 g of oleic acid and an alkaline catalyst were added, and esterification reaction was carried out at 260°C. The reaction was continued until the acidic value of a reactant achieved 0.5 or less, to provide a polyglycerol fatty acid ester of Example 1. Other Examples and Comparative Examples were also prepared according to the same procedure. In Examples 10 to 14 and Comparative Examples 8 to 11, esterification reaction was carried out at 240°C. Incidentally, the HLB of the polyglycerol fatty acid ester

can be adjusted by changing the ratio of polyglycerol and fatty acid. For example, reaction and preparation were carried out using 24.2 g of polyglycerol and 15.8 g of oleic acid in the case of Example 6 (HLB 11.9), 22.1 g of polyglycerol and 17.9 g of oleic acid in the case of Example 7 (HLB 10.8), and 18.4 g of polyglycerol and 21.6 g of oleic acid in the case of Example 8 (HLB 8.8).

< Test Example 1: Temperature Stability>

Polyglycerol fatty acid esters of Examples 1 to 14, 16 to 17 and Comparative Examples 1 to 11, 13 to 15 were heated and dissolved in oil agents described in Tables 2 to 4 at 70°C so as to have concentrations (5% by mass and 15% by mass) described in Tables 2 to 4. The dissolved compositions were placed into a vial bottle (volume 20 ml, inner diameter 2.5 cm) in an amount of 10 g, and stored at -5°C, 25°C and 50°C for 1 month, and thereafter the appearance properties were visually observed according to the following evaluation criteria. The results are given in Tables 2 to 4.

(Evaluation Criteria)

○: No turbidity and precipitation are found
Δ: Slight turbidity is found
×: Precipitated

Details of components described in Tables 2 to 4 are shown below.

Cetyl ethylhexanoate: EXCEPARL HO / Kao Corporation
Glyceryl tri(caprylate/caprate): SUNOIL MCT-7 / Taiyo Kagaku Co., Ltd.
Mineral oil: MORESCOWHITE P-70 / MORESCO Corporation

[Table 2]

Table 2

| | Polyglycerol used | Fatty acid | HLB | Temperature stability Concentration: 5% Oil agent: cetyl ethylhexanoate | | | Temperature stability Concentration: 5% Oil agent: glyceryl tri(caprylate/caprate) | | | Temperature stability Concentration: 5% Oil agent: mineral oil | | | Temperature stability Concentration: 15% Oil agent: cetyl ethylhexanoate | | |
|---|---|---|---|---|---|---|---|---|---|---|---|---|---|---|---|
| | | | | -5°C | 25°C | 50°C | -5°C | 25°C | 50°C | -5°C | 25°C | 50°C | -5°C | 25°C | 50°C |
| Ex. 1 | Polyglycerol 1 | Oleic acid | 9.8 | ○ | ○ | ○ | ○ | ○ | ○ | ○ | ○ | ○ | ○ | ○ | ○ |
| Ex. 2 | Polyglycerol 2 | Oleic acid | 9.8 | ○ | ○ | O | ○ | ○ | ○ | ○ | ○ | ○ | ○ | ○ | ○ |
| Ex. 3 | Polyglycerol 3 | Oleic acid | 9.8 | ○ | ○ | ○ | ○ | ○ | ○ | ○ | ○ | ○ | ○ | ○ | ○ |
| Ex. 4 | Polyglycerol 4 | Oleic acid | 9.8 | ○ | ○ | ○ | ○ | ○ | ○ | ○ | ○ | ○ | ○ | ○ | ○ |
| Ex. 5 | Polyglycerol 5 | Oleic acid | 9.8 | △ | O | ○ | △ | O | ○ | △ | ○ | ○ | ○ | ○ | ○ |
| Comp. Ex. 1 | Polyglycerol 6 | Oleic acid | 9.8 | × | △ | ○ | × | ○ | ○ | × | △ | ○ | × | ○ | ○ |
| Comp. Ex. 2 | Polyglycerol 7 | Oleic acid | 9.8 | × | × | △ | × | × | × | × | × | × | × | △ | ○ |
| Comp. Ex. 3 | Polyglycerol 8 | Oleic acid | 9.8 | × | × | △ | × | × | × | × | × | × | × | △ | ○ |
| Comp. Ex. 13 | Polyglycerol 9 | Oleic acid | 9.8 | × | △ | ○ | × | △ | O | × | △ | ○ | × | ○ | ○ |
| Ex. 16 | Polyglycerol 10 | Oleic acid | 9.8 | ○ | ○ | ○ | ○ | ○ | ○ | ○ | ○ | ○ | ○ | ○ | ○ |
| Ex. 17 | Polyglycerol 11 | Oleic acid | 9.8 | ○ | ○ | ○ | ○ | ○ | ○ | ○ | ○ | ○ | ○ | ○ | ○ |
| Comp. Ex. 14 | Polyglycerol 12 | Oleic acid | 9.8 | × | × | ○ | × | × | ○ | × | × | ○ | × | △ | ○ |
| Comp. Ex. 15 | Polyglycerol 13 | Oleic acid | 9.8 | × | × | ○ | × | × | ○ | × | × | ○ | × | △ | ○ |

[Table 3]

Table 3

| | Polyglycerol used | Fatty acid | HLB | Temperature stability Concentration: 5% Oil agent: cetyl ethylhexanoate | | | Temperature stability Concentration: 15% Oil agent: cetyl ethylhexanoate | | |
|---|---|---|---|---|---|---|---|---|---|
| | | | | -5°C | 25°C | 50°C | -5°C | 25°C | 50°C |
| Ex. 6 | Polyglycerol 2 | Oleic acid | 11.9 | ○ | ○ | ○ | ○ | ○ | ○ |
| Ex. 7 | Polyglycerol 2 | Oleic acid | 10.8 | ○ | ○ | ○ | ○ | ○ | ○ |
| Ex. 8 | Polyglycerol 2 | Oleic acid | 8.8 | ○ | ○ | ○ | ○ | ○ | ○ |
| Ex. 9 | Polyglycerol 2 | Isostearic acid | 9.8 | ○ | ○ | O | ○ | ○ | ○ |
| Comp. Ex? 4 | Polyglycerol 8 | Oleic acid | 11.9 | × | × | × | × | × | × |
| Comp. Ex. 5 | Polyglycerol 8 | Oleic acid | 10.8 | × | × | × | × | × | × |
| Comp. Ex. 6 | Polyglycerol 8 | Oleic acid | 8.8 | × | △ | △ | × | ○ | ○ |
| Comp. Ex. 7 | Polyglycerol 8 | Isostearic acid | 9.8 | × | × | × | × | △ | ○ |

[Table 4]

Table 4

| | Polyglycerol used | Fatty acid | HLB | Temperature stability Concentration: 5% Oil agent: cetyl ethylhexanoate | | | Temperature stability Concentration: 15% Oil agent: cetyl ethylhexanoate | | |
|---|---|---|---|---|---|---|---|---|---|
| | | | | -5°C | 25°C | 50°C | -5°C | 25°C | 50°C |
| Ex. 10 | Polyglycerol 2 | capric acid | 10.2 | Δ | △ | ○ | ○ | ○ | ○ |
| Ex. 11 | Polyglycerol 3 | capric acid | 10.2 | Δ | Δ | △ | ○ | ○ | ○ |
| Ex. 12 | Polyglycerol 2 | caprylic acid | 10.2 | △ | Δ | △ | ○ | ○ | ○ |
| Ex. 13 | Polyglycerol 2 | isononanoic acid | 10.2 | △ | Δ | △ | ○ | ○ | ○ |
| Ex. 14 | Polyglycerol 2 | ratio of caprylic acid and lauric acid (1:1) | 10.2 | △ | ○ | ○ | ○ | ○ | ○ |
| Comp. Ex 8 | Polyglycerol 8 | capric acid | 10.2 | × | × | × | × | × | × |

(continued)

| | Polyglycerol used | Fatty acid | HLB | Temperature stability Concentration: 5% Oil agent: cetyl ethylhexanoate | | | Temperature stability Concentration: 15% Oil agent: cetyl ethylhexanoate | | |
|---|---|---|---|---|---|---|---|---|---|
| | | | | -5°C | 25°C | 50°C | -5°C | 25°C | 50°C |
| Comp. Ex 9 | Polyglycerol 8 | caprylic acid | 10.2 | × | × | × | × | × | × |
| Comp. Ex 10 | Polyglycerol 8 | isononanoic acid | 10.2 | × | × | × | × | × | × |
| Comp. Ex 11 | Polyglycerol 8 | ratio of caprylic acid and lauric acid (1:1) | 10.2 | × | × | × | × | × | × |

Example 15, and Comparative Example 12

Compositions were prepared in the proportions shown in Table 5, to provide Example 15 and Comparative Example 12. Each of temperature stability, particle size upon washing out and eye irritability was evaluated. The temperature stability was evaluated in the same manner as Test Example 1. The results are given in Table 5.

< Test Example 2: Emulsified Particle Size upon Washing Out >

Compositions of Example 15 and Comparative Example 12 were heated and dissolved in cetyl ethylhexanoate at 70°C to have a concentration of 15% by mass. After cooling to an ambient temperature, 1 g of the compositions were placed into a vial bottle (volume 100 ml, inner diameter 3.2 cm). Water was added thereto in an amount of 99 g and mixed, and then particle size distribution was measured with a particle size analyzer (BECKMAN COULTER LS 13 320). The measured average particle sizes were considered as emulsified particle sizes upon washing out. The results are given in Table 5.

< Test Example 3: Eye Irritability >

Compositions of Example 15 and Comparative Example 12 were heated and dissolved in cetyl ethylhexanoate at 70°C to have a concentration of 15% by mass. After cooling to an ambient temperature, when the compositions were applied to the eye area, eye irritability was evaluated according to the following evaluation criteria. Five professional panelists carried out the evaluation, and the average was calculated. The results are given in Table 5.

(Evaluation Criteria)

1: Eyes are not irritable

2: Eyes are slightly irritable

3: Eyes are irritable

[Table 5]

Table 5

| | Proportion of composition (%) | | Temperature stability Concentration: 5% Oil agent: cetyl ethylhexanoate | | | Temperature stability Concentration: 15% Oil agent: cetyl ethylhexanoate | | | Emulsified particle size upon washing out ($\mu$m) | Eye irritability |
|---|---|---|---|---|---|---|---|---|---|---|
| | | | -5°C | 25°C | 50°C | -5°C | 25°C | 50°C | | |
| Ex. 15 | Ex. 2 | 30 | ○ | ○ | ○ | ○ | ○ | ○ | 0.325 | 1.6 |
| | Ex. 10 | 70 | | | | | | | | |
| Com. Ex. 12 | Comp. Ex. 2 | 30 | × | × | × | × | × | ○ | 1.276 | 2.2 |
| | Comp. Ex. 7 | 70 | | | | | | | | |

As shown in Tables 2 to 5, all of Examples 1 to 17 using the polyglycerol fatty acid ester of the present invention had excellent temperature stability in wide temperature range including low temperature such as -5°C. In addition, as shown in Table 5, the polyglycerol fatty acid ester can be suitably used in various cosmetic applications, because the polyglycerol fatty acid ester has a small emulsified particle size upon washing out and lower eye irritability.

INDUSTRIAL APPLICABILITY

According to the present invention, a polyglycerol fatty acid ester which is excellent in solubility to an oil agent in wide temperature range can be provided, and the polyglycerol fatty acid ester can be suitably used in an application such as cosmetics.

## Claims

1. A polyglycerol fatty acid ester consisting of a polyglycerol of which the ratio of a branched glycerol group represented by the following formula (1):

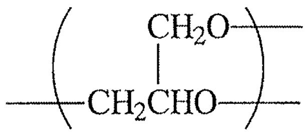

(1)

is from 14 to 36% and an average degree of polymerization is from 5 to 22, and a fatty acid having the number of carbon atoms of from 6 to 22, wherein the total content of glycerol and diglycerol in the polyglycerol is from 2 to 20% by mass.

2. The polyglycerol fatty acid ester according to claim 1, wherein an HLB is from 8 to 13.

3. The polyglycerol fatty acid ester according to claim 1, wherein the number of the branched glycerol group per polyglycerol molecule is from 1 to 10 on average.

4. The polyglycerol fatty acid ester according to claim 1, wherein the fatty acid is isostearic acid or oleic acid.

5. The polyglycerol fatty acid ester according to claim 1, wherein the fatty acid is caprylic acid, isononanoic acid or capric acid.

6. A cosmetic composition comprising the polyglycerol fatty acid ester as defined in any of claims 1 to 5 and an oil agent.

7. Cosmetics comprising the cosmetic composition as defined in claim 6.

[FIG. 1]

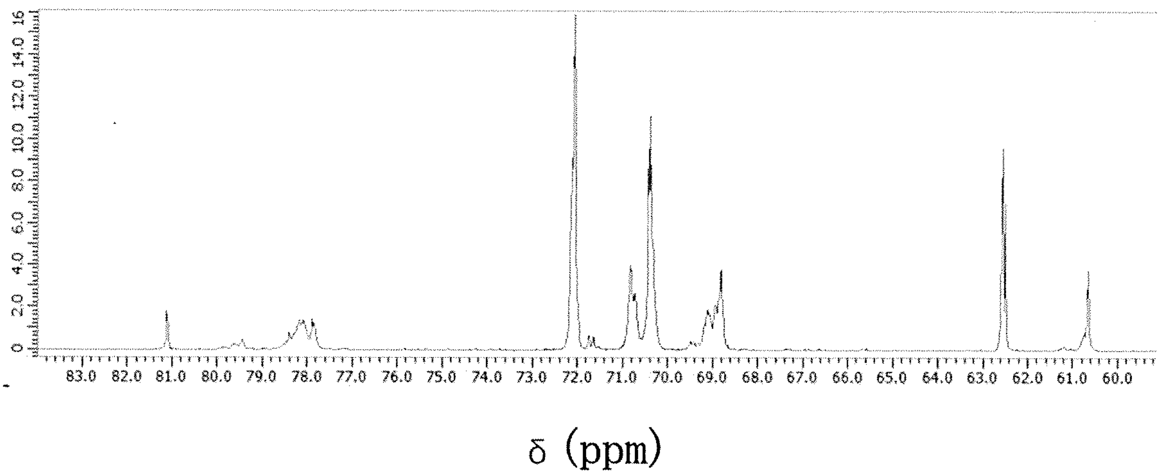
0
2.0
4.0
6.0
8.0
10.0
12.0
14.0
16
83.0 82.0 81.0 80.0 79.0 78.0 77.0 76.0 75.0 74.0 73.0 72.0 71.0 70.0 69.0 68.0 67.0 66.0 65.0 64.0 63.0 62.0 61.0 60.0
δ (ppm)

## INTERNATIONAL SEARCH REPORT

International application No. **PCT/JP2023/038339**

### A. CLASSIFICATION OF SUBJECT MATTER

***A61K 8/86***(2006.01)i; ***A61K 8/37***(2006.01)i; ***A61K 8/39***(2006.01)i; ***A61Q 19/10***(2006.01)i; ***C07C 69/33***(2006.01)i; ***C08G 65/332***(2006.01)i
FI: A61K8/86; A61K8/39; A61Q19/10; A61K8/37; C08G65/332; C07C69/33 CSP

According to International Patent Classification (IPC) or to both national classification and IPC

### B. FIELDS SEARCHED

Minimum documentation searched (classification system followed by classification symbols)

A61K8/86; A61K8/37; A61K8/39; A61Q19/10; C07C69/33; C08G65/332

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2006-346526 A (TAIYO KAGAKU CO., LTD.) 28 December 2006 (2006-12-28) entire text | 1-7 |
| A | JP 2007-209251 A (TAIYO KAGAKU CO., LTD.) 23 August 2007 (2007-08-23) entire text | 1-7 |
| A | JP 2001-064236 A (TAIYO KAGAKU CO., LTD.) 13 March 2001 (2001-03-13) entire text | 1-7 |
| A | JP 2006-213698 A (RIKEN VITAMIN CO., LTD.) 17 August 2006 (2006-08-17) entire text | 1-7 |
| A | JP 9-238619 A (TAIYO KAGAKU CO., LTD.) 16 September 1997 (1997-09-16) entire text | 1-7 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/038339**

| Patent document cited in search report | Publication date (day/month/year) | Patent family member(s) | Publication date (day/month/year) |
|---|---|---|---|
| JP 2006-346526 A | 28 December 2006 | US 2009/0018358 A1<br>entire text<br>EP 1801096 A1 | |
| JP 2007-209251 A | 23 August 2007 | (Family: none) | |
| JP 2001-064236 A | 13 March 2001 | US 2002/0035238 A1<br>entire text | |
| JP 2006-213698 A | 17 August 2006 | EP 1679300 A1<br>entire text | |
| JP 9-238619 A | 16 September 1997 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7173380 A